# EUROPÄISCHE PATENTSCHRIFT

(11) **EP 0 900 558 B1**
(45) Veröffentlichungstag und Bekanntmachung des Hinweises auf die Patenterteilung: **02.07.2003**
(21) Anmeldenummer: 98116362.9
(22) Anmeldetag: 28.08.1998
(51) Int. Cl.: A61K 7/00, A61K 7/06

(54) **Haarpflegemittel**
Haircare product
Agent pour le soin du cheveu

(30) Priorität: 02.09.1997 DE 19738247
(43) Veröffentlichungstag der Anmeldung: 10.03.1999
(73) Patentinhaber: KPSS-Kao Professional Salon Services GmbH, 64297 Darmstadt (DE)
(72) Erfinder: Dubowoj, Polina, 64319 Pfungstadt (DE); Uellner, Martin, 64297 Darmstadt (DE)

(56) Entgegenhaltungen:
- EP-A- 0 318 369
- EP-A- 0 376 852
- EP-A- 0 412 865
- EP-A- 0 452 202
- EP-A- 0 531 192
- US-A- 5 034 228

## Beschreibung

Die vorliegende Erfindung betrifft ein Haarpflegemittel mit haarkonditionierenden Eigenschaften.
Solche Mittel sind seit langem bekannt und erfolgreich auf dem Markt.
Dabei werden, neben polymeren haarkonditionierenden Substanzen, auch öl- bzw. fettartige synthetische und natürliche Wirkstoffe benutzt.
Die Einarbeitung dieser und anderer Wirkstoffe in wäßrige Grundlagen erweist sich jedoch häufig als schwierig, da es hierfür grundsätzlich der Emulsionsform bedarf, was wiederum Stabilitätsprobleme hervorrufen kann. Eine Einarbeitung in transparente bzw. transluzente Grundlagen auf wäßriger Basis, enthaltend Verdickungsmittel, ist in der Regel gar nicht möglich.

Aus der EP 590 538 B1 sind bereits durchsichtige Haarpflegemitttel bekannt, die in einer wäßrigen, gelförmigen Grundlage ein Suspendierhilfsmittel und Mikrokapseln enthalten, in die in ein wasserunlösliches konditionierendes Material eingekapselt ist.
Bei der Anwendung sollen diese Mikrokapseln zerstört und in Teilchen mit einen Maximaldurchmesser von 10 Mikron zerkleinert werden, um Rückstände im Haar zu vermeiden.
Dies funktioniert jedoch nur bedingt, bei Anwendung dieser bekannten Zusammensetzung lassen sich Rückstände im Haar, bestehend aus dem Kapselwandmaterial, nicht vermeiden, es wird darüber hinaus auch keine homogene Verteilung des eingekapselten Wirkstoff auf dem Haar erzielt.

Die Europäischen Patentanmeldungen EP 452 202 A1, EP 412 865 A1 und EP 531 192 A1 beschreiben Mikropartikel enthaltende, gelartige Zusammensetzungen, die auch zur Haarpflege benutzt werden können. Die in den genannten Dokumenten beschriebenen Mikropartikel bestehen aus fetthaltigen Stoffen, solche Mikropartikel werden auch in der EP 376 852 A1 beschrieben. Die in diesen Dokumenten beschriebenen Mikropartikel können sowohl pharmazeutisch als auch kosmetisch wirkende Inhaltsstoffe, wie UV-Filter oder Duftstoffe, enthalten.

Die Erfindung geht daher von der Aufgabenstellung aus, ein konditionierendes Haarpflegemittel auf Basis eines wäßrigen Gels zu entwickeln, das die obengenannten Nachteile der bekannten Produkte nicht aufweist.

Diese Aufgabe wird durch ein Haarpflegemittel auf Basis einer wäßrigen, gelförmigen Grundlage, enthaltend mindestens ein Verdickungsmittel und gegebenenfalls mindestens einen Emulgator sowie 0,25 bis 50 Gew.-%, berechnet auf die Gesamtzusammensetzung des Mittels, an zur Farbe der wäßrigen Grundlage in farblichem Kontrast stehende Mikropartikel, die mindestens eine öl- bzw. fettartige konditionierende Substanz sowie Agar-Agar und Alginsäure enthalten, wobei diese Mikropartikel im wesentlichen eine durchgehend homogene Struktur (d.h., keine Kapselwand) besitzen und einen durchschnittlichen mittleren Teilchendurchmesser von 0,1 bis 4 mm aufweisen, gelöst.

Diese Zusammensetzungen verteilen sich homogen und rückstandsfrei auf dem Haar, da von den Mikroteilchen keine Kapselwandrückstände vorliegen können, insbesondere bei Benutzung einer Verpackung, die in zylindrischer oder ovaler Form gestaltet ist und einen nach oben beweglichen Kolben und einen Verschlußkopf aufweist, der mit dem zylindrischen Verpackungskörper über einen elastischen, zusammendrückbaren Balg an seinem unteren Ende mit einem Ventil verbunden ist und an seinem oberen Ende in ein Abgaberohr mündet, das in einer mit einer Membran versehenen Ausgabeöffnung endet, oder bei Verwendung einer maunuell zu betätigenden Pumpvorrichtung, die auf einen Behälter aufgebracht wird, und einen Schaft umfaßt, der an seinem unteren Ende in ein Steigrohr und an seinem oberen Ende in einen Druckknopf mündet, bei dessen Betätigung der Behälterinhalt über eine Ausgabeöffnung abgegeben wird, und die eine Feder aufweist, wobei diese Betätigungsvorrichtung in eine Schraub- oder Aufsteckkappe integriert ist, die an ihrer Innenseite Mittel zur Befestigung auf einem Behälter aufweist.

Darüberhinaus wird, bei Verwendung eines transparenten Behälters, auch noch ein den Verbraucher ansprechender, ästhetischer Effekt erzielt.

Die erfindungsgemäßen Mittel lassen sich besonders günstig verteilen, wenn die Viskosität der Gel-Grundlage zwischen 2500 bis 75 000 mPa.s, insbesondere zwischen 5000 bis 50 000 mPa.s, gemessen im Brookfield-Viskosimeter (Spindel Nr. 5) bei 20°C und 5 rpm liegt.

Der pH-Wert liegt vorzugsweise bei 3,5 bis 6,5.

Die erfindungsgemäß verwendete Gel-Grundlage enthält im Regelfall etwa 0,1 bis etwa 2,5 Gew.-% eines Verdickungsmittels, das gleichzeitig auch als Suspendierhilfsmittel wirkt.

Grundsätzlich ist hierfür jedes in der Kosmetik übliche und bekannte Verdickungsmittel geeignet, beispielsweise Cellulosederivate wie Hydroxyethyl- oder Hydroxypropylmethylcellulose, natürliche Gummen wie Guar Gum, Xanthan Gum, Karaya Gum, Tragant, Alginate, Dextrine, Carrageen, synthetische Polymere, insbesondere Polyacrylsäure und deren Salze vom Typ "Carbomer" oder Polyvinylpyrrolidon und gegebenenfalls auch anorganische Substanzen wie Monmorillonite und Silikagele.
Fett- und/oder ölartige konditionierende Mittel, die in den erfindungsgemäß eingesetzten Mikroteilchen enthalten sind, umfassen synthetische und natürliche Fette, Öle und fett- und ölartige Substanzen.
Geeignete Fette und Öle, zu denen auch Wachse zählen, sind insbesondere natürliche pflanzliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl oder auch Oliven- bzw. Sojaöl, Lanolin und dessen Derivate, ebenso Mineralöle wie Paraffinöl und Vaseline.
Synthetische Öle und Wachse sind insbesondere Silikonöle, wie die bekannten Polysiloxane, Polyethylenglykole, etc.
Natürliche Wachse sind beispielsweise niedrigschmelzende Paraffinwachse, Orangenwachs, Apfelwachs, etc.

Weitere geeignete konditionierende Komponenten sind Fettalkohole, vorzugsweise solche mit etwa 8 bis 22 Kohlenstoffatomen im Molekül wie Myristyl-, Cetyl-, Stearylalkohol, Wachsalkohole und Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Cetylpalmitat, Polyethylenglykol- und Polyglycerylfettsäureester wie PEG-7-glycerylcocoat, , etc. Diese Aufzählung ist nur beispielhaft und erhebt keinerlei Anspruch auf Vollständigkeit.

Der bevorzugte mittlere Teilchendurchmesser der Mikroteilchen liegt zwischen etwa 0,5 und 2,5 mm, insbesondere bei 0,75 bis 2 mm.
Sie stehen in farblichem Kontrast zu der wäßrigen Grundlage, so daß sie für den Verbraucher in der Verpackung sichtbar sind und einen optischen Anreiz ausüben.

Gemäß einer bevorzugten Ausführungsform der Erfindung enthalten die Mikropartikel, zusätzlich zu den haarkonditionierenden Substanzen, UV-Absorber, beispielsweise Benzophenone-1, Benzophenone-2, Benzophenone-3, Benzophenone-4, Benzophenone-5, Benzophenone-6, Benzophenone-8, Benzophenone-9, Benzophenone-10, Benzophenone-12, 4-Isopropyldibenzoylmethan, 3-(4-Methylbenzoyliden)campher, Octylmethoxycinnemat, 2-Ethylhexylmethoxycinnamat, 1-(4-Methoxyphenol)-3(4-tert.-butylphenyl)propan-1.3-dion, Salicylsäureester, Phenylbenzimidazolsulfonsäure, etc.

Die erfindungsgemäßen Zusammensetzungen können, innerhalb und außerhalb der Mikroteilchen, alle in Haarpflegemitteln üblichen Wirk- und Hilfsstoffe enthalten, sofern sie die Stabilität und Kompatibilität dieser Produkte nicht beeinträchtigen.

Zur Vermeidung von Wiederholungen wird auf den Stand der Technik, zusammengefaßt beispielsweise in der Monographie von K. Schrader, Grundlagen und Rezepturen der Kosmetika, 2. Aufl. (1989, Hüthig Buch Verlag), S. 676-771, verwiesen.

Die erfindungsgemäß verwendeten Mikroteilchen enthalten, neben mindestens einer öl- bzw. fettartigen haarkonditionierenden Substanz und gegebenenfalls einen UV-Absorber vorzugsweise noch mindestens einen Emulgator, vorzugsweise ein nichtionisches Tensid.

Geeignet sind insbesondere C₈-C₂₀-Alkylpolyglucoside mit einem Polykondensationsgrad von 1 bis 5, insbesondere 1,2 bis 2,5, Polyoxyethylensorbitanfettsäureester wie PEG-4-oder PEG-20-sorbitanmonolaurat, -monostearat, -monopalmitat, -monooleat, -tristearat oder -trioleat, Polyoxyethylenfettalkoholether, langkettige Aminoxide wie Lauryldimethylaminoxid, etc., deren bevorzugter Anteil bei 0,1 bis 10, vorzugsweise 0,25 bis 5 Gew.-%, bezogen auf das Gewicht der Mikroteilchen, liegt.
Selbstverständlich kann auch die Grundlage Emulgatoren enthalten, was dem Fachmann geläufig ist.

Falls das erfindungsgemäße Gel eine Styling-Gel oder Haarfixativ darstellt, enthält es vorzugsweise ein oder mehrere synthetische oder natürliche haarfestigende Polymere. Diese sind dem Fachmann ebenfalls bekannt und beispielsweise bei K. Schrader, l.c., beschrieben

Die Herstellung der durchweg eine homogene Verteilung aufweisenden Mikropartikel erfolgt durch den Einschluß des hydrophoben, öl- bzw. fettartigen Materials mittels Liposomen in einem Mikrofluidizer in Form einer kolloidalen hydrophilen Matrix; diese Teilchen sind in wäßrigem Medium bis zur Zerstörung stabil.
Geeignetes Material für diese Liposome sind insbesondere Phospholipide pflanzlichen Ursprungs, die gleichzeitig als Emulgatoren wirken.
Andererseits können Emulsionen enthaltende, eine homogene Verteilung aufweisende Mikroteilchen auch durch Integration in ein Alginsäure/Agar Agar-Material dargestellt werden.

Die entsprechenden Verfahren sind dem Fachmann bekannt (vgl. beispielsweise auch F. Zülli, F. Suter, Proceedings Intern. Symp. Control Rel. Bioact. Materials, Vol.21 (1994), S. 459-460) und bedürfen hier daher keiner weiteren Beschreibung.

Im folgenden werden einige Beispiele für erfindungsgemäße Zusammensetzungen gegeben.

### Beispiel 1

| **Leave-on-Gel** | |
|---|---|
| Polyacrylsäure (Carbopol^{R}2020) | 0,5 (Gew.-%) |
| 1,2-Propandiol | 2,0 |
| NaOH (32%) | 0,1 |
| Ethanol | 20,0 |
| Polysorbate-60 | 0,5 |
| Dimethicone Copolyol | 1,5 |
| Parfum, Konservierungsmittel | q.s. |
| Mikropartikel^{x} (mittl. Teilchendurchmesser 1,5mm) | 2,5 |
| Wasser | ad 100,00 |

| | |
|---|---|
| ^{**x**}**Zusammensetzung:** | |

| | |
|---|---|
| Mandelöl | 20,0 (Gew.-%) |
| Glycerin | 10,0 |
| Talkum | 5,0 |
| Ti0₂ | 1,0 |
| Agar-Agar | 1,0 |
| Alginsäure | 0,5 |
| Carboxymethylcellulose | 0,3 |
| Polysorbate-20 | 0,2 |
| Farbstoff (C.I.-No. 77491) | 0,1 |
| Konservierungsmittel | 0,5 |
| Wasser | ad 100,0 |

Die pinkfarbenen Mikropartikel verschwanden rückstandsfrei nach dem Aufbringen auf das Haar mittels einer geeigneten, eingangs beschriebenen Abgabevorrichtung. Mit dem Produkt wird eine ausgezeichnete haarkonditionierende Wirkung erzielt

### Beispiel 2

| **Konditionierendes und pflegendes Gel** | |
|---|---|
| Polyacrylsäure (Carbopol^{R}2050) | 0,8 (Gew.-%) |
| NaOH | 0,3 |
| PEG-60-Hydriertes Ricinusöl | 0,3 |
| Dimethicone Copolyol | 3,0 |
| Benzyloxyethanol | 0,3 |
| 1-2-Propylenglykol | 3,0 |
| Quaterniertes Weizenproteinhydrolysat (Gluadin^{R}W40) | ,05 |
| Ethanol | 25,0 |
| Parfum, Konservierungsmittel | q.s. |
| Mikroteilchen^{x} (mittl. Teilchendurchmesser 2mm) | 6,5 |
| Wasser | ad 100,0 |

| | |
|---|---|
| ^{**x**}**Zusammensetzung der Mikroteilchen:** | |

| | |
|---|---|
| Weizenkeimöl | 20,0 (Gew.-%) |
| Glycerin | 10,0 |
| Agar Agar | 1,0 |
| Polysorbate-20 | 0,55 |
| Alginsäure | 0,50 |
| Carboxymethylcellulose | 0,30 |
| Konservierungsmittel | 0,50 |
| Farbstoff (C.I.-No. 77 491) | 0,05 |
| Farbstoff (C.I.-No. 77 492) | 0,50 |
| Wasser | ad 100,0 |

Es wurde ein Gel mit orangefarbenen Mikropartikeln erhalten, das auf dem Haar Glanz, Volumen, weichen Griff und Elastizität ohne iregendwelche störenden Rückstände ergab.

## Patentansprüche

1. Haarpflegemittel auf Basis einer wäßrigen gelförmigen Grundlage, enthaltend mindestens ein Verdickungsmittel sowie 0,25 bis 50 Gew.-%, berechnet auf die Gesamtzusammensetzung, an zur Farbe der wäßrigen Grundlage in farblichem Kontrast stehenden Mikropartikeln, wobei diese Mikropartikel im wesentlichen eine durchgehend homogene Struktur und einen durchschnittlichen mittleren Teilchendurchmesser von 0,1 bis 4 mm aufweisen **dadurch gekennzeichnet, daß die Mikropartikel mindestens eine öl- bzw. fettartige konditionierende Substanz, Agar Agar und Alginsäure enthalten.**

2. Haarpflegemittel nach Anspruch 1, **dadurch gekennzeichnet, daß** die wäßrige gelförmige Grundlage eine Viskosität von 2500 bis 75 000 mPa.s, gemessen bei 20°C im Brookfield-Viskosimeter bei 5 rpm, aufweist.

3. Haarpflegemittel nach Anspruch 2, **dadurch gekennzeichnet, daß** die wäßrige gelförmige Grundlage eine Viskosität von 5000 bis 50 000 mPa.s, gemessen bei 20°C im Brookfield-Viskosimeter bei 5 rpm, aufweist.

4. Haarpflegemittel nach einem oder mehreren der Ansprüche 1 bis 3, **gekennzeichnet durch** einen pH-Wert von 3,5 bis 6,5.

5. Haarpflegemittel nach einem oder mehreren der Ansprüche 1 bis 4, **dadurch gekennzeichnet, daß** die Mikropartikel mindestens einen UV-Absorber enthalten,

6. Haarpflegemittel nach einem oder mehreren der Ansprüche 1 bis 5, **dadurch gekennzeichnet, daß** dieMikropartikel mindestens einen Emulgator enthalten.

## Claims

1. Hair treatment composition on the basis of an aqueous, gel-like carrier, comprising at least one thickening agent as well as 0.25% to 50% by weight, calculated to the total composition, of microparticles of a color contrasting to that of the aqueous carrier, whereby these microparticles have an essentially homogeneous structure and an average particle diameter of 0.1 to 4 mm, **characterized in that** these microarticles comprise at least one fatty or oily conditioning compound, agar-agar and alginic acid.

2. Hair treatment composition according to claim 1, wherein the aqueous, gel-like carrier has a viscosity of 2,500 to 75,000 mPa.s, measured at 20C° in the Brookfield Viscosimeter at 5 rpm.

3. Hair treatment composition according to claim 2, wherein the aqueous, gel-like carrier has a viscosity of 5,000 to 50,000 mPa.s, measured at 20C° in the Brookfield Viscosimeter at 5 rpm.

4. Hair treatment composition according to on or more of claims 1 to 3, having a pH-value of 3.5 to 6.5.

5. Hair treatment composition according to one or more of claims 1 to 4, wherein the microparticles contain at least one UV-absorber.

6. Hair treatment composition according to one or more of claims 1 to 5, wherein the microparticles contain at least one emulsifier.

## Revendications

1. Agent de soins capillaires à base de fond aqueux sous forme de gel, contenant au moins un épaississant ainsi que 0,25 à 50% en poids, calculés par rapport à la composition totale, de microparticules se trouvant en contraste sur le plan des couleurs pour colorer le fond aqueux, ces microparticules présentant essentiellement une structure homogène en continu et un diamètre de particule médian moyen de 0,1 à 4 mm, **caractérisé en ce que** les microparticules contiennent au moins une substance de conditionnement analogue à une huile ou matière grasse, de l'agar-agar et de l'acide alginique.

2. Agent de soins capillaires selon la revendication 1, **caractérisé en ce que** le fond aqueux sous forme de gel présente une viscosité de 2 500 à 75 000 mPa.s, mesurée à 20°C dans le viscosimètre Brookefield à 5 tr/mn.

3. Agent de soins capillaires selon la revendication 2, **caractérisé en ce que** le fond aqueux sous forme de gel présente une viscosité de 5 000 à 50 000 mPa.s, mesurée à 20°C dans le viscosimètre Brookefield à 5 tr/mn.

4. Agent. de soins capillaires selon l'une quelconque ou plusieurs des revendications 1 à 3, **caractérisé par** une valeur de pH de 3,5 à 6,5.

5. Agent de soins capillaires selon l'une quelconque ou plusieurs des revendications 1 à 4, **caractérisé en ce que** les microparticules contiennent au moins un absorbeur U.V.

6. Agent de soins capillaires selon l'une quelconque ou plusieurs des revendications 1 à 5, **caractérisé en ce que** les microparticules contiennent au moins un émulsifiant.
